(19) Europäisches Patentamt
European Patent Office
Office européen des brevets

(11) Veröffentlichungsnummer: **0 137 452**
**B1**

(12) # EUROPÄISCHE PATENTSCHRIFT

(45) Veröffentlichungstag der Patentschrift: **13.09.89**

(51) Int. Cl.⁴: **C 07 D 251/24**, G 03 F 7/10, C 07 D 405/10

(21) Anmeldenummer: **84111892.0**

(22) Anmeldetag: **04.10.84**

(54) **Lichtempfindliche, Trichlormethylgruppen aufweisende Verbindungen, Verfahren zu ihrer Herstellung und diese Verbindungen enthaltendes lichtempfindliches Gemisch.**

(30) Priorität: **12.10.83 DE 3337024**

(43) Veröffentlichungstag der Anmeldung: **17.04.85 Patentblatt 85/16**

(45) Bekanntmachung des Hinweises auf die Patenterteilung: **13.09.89 Patentblatt 89/37**

(84) Benannte Vertragsstaaten: **AT BE CH DE FR GB IT LI NL SE**

(56) Entgegenhaltungen:
**DE-A-2 243 621**
**DE-A-2 718 259**
**US-A-3 758 462**

(73) Patentinhaber: **HOECHST AKTIENGESELLSCHAFT, Postfach 80 03 20, D-6230 Frankfurt am Main 80 (DE)**

(72) Erfinder: **Buhr, Gerhard, Dr. Dipl.- Chem., Am Erdbeerstein 28, D-6240 Königstein/Ts. (DE)**

**EP 0 137 452 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft Bis-4,6-trichlormethyl-s-triazine, die in 2-Stellung durch einen aromatischen Rest substituiert sind, sowie ein lichtempfindliches Gemisch, das diese Verbindungen enthält.

Verbindungen der genannten Gattung sind als Initiatoren für verschiedene photochemische Reaktionen bekannt. Sie werden einerseits eingesetzt, um die bei Einwirkung von aktinischer Strahlung entstehenden Radikale zur Auslösung von Polymerisationsreaktionen oder Farbveränderungen auszunutzen, oder andererseits, um durch die freigesetzte Säure Folgereaktionen zu bewirken.

In der DE-A-2 243 621 werden styrylsubstituierte Trichlormethyl-s-triazine bechrieben, die eine Reihe von vorteilhaften Eigenschaften haben. Nachteilig ist ihre relativ komplizierte Herstellung.

In der DE-A-2 718 259 werden 2-Aryl-4,6-bis-trichlormethyl-s-triazine mit mehrkernigen Arylgruppen beschrieben, die ähnlich vorteilhafte Eigenschaften, insbesondere eine hohe Lichtempfindlichkeit, aufweisen und die sich auf einfache Weise herstellen lassen. Sie haben jedoch in der Regel die hohe Empfindlichkeit nur in einem Spektralbereich und lassen sich deshalb nicht zu lichtempfindlichen Materialen verarbeiten, die gegenüber unterschiedlichen Lichtquellen, z. B. Argon-Ionen-Lasern und galliumdotierten Quecksilberdampflampen, eine gleich hohe Empfindlichkeit aufweisen.

Man hat auch bereits versucht, Photopolymerisationsinitiatoren anderer Struktur mit solchen zu kombinieren, die Trichlormethylgruppen enthalten, um verschiedene angestrebte Bigenschaften in einer Mischung zu verwirklichen (DE-A-2 851 641).

Aufgabe der Erfindung war es, Photoinitiatoren vorzuschlagen, die sich in einfacher Weise herstellen lassen und die in ihrer Empfindlichkeit gegenüber der UV -Strahlung eines Argon-Ionen-Lasers einerseits und der Strahlung einer galliumdotierten Quecksilberdampf-Hochdrucklampe im sichtbaren Spektralbereich andererseits jeweils mindestens den Photoinitiatoren entsprechen, die in dem jeweiligen Spektralbereich besonders empfindlich sind.

Gegenstand der Erfindung sind lichtempfindliche Verbindungen der allgemeinen Formel I:

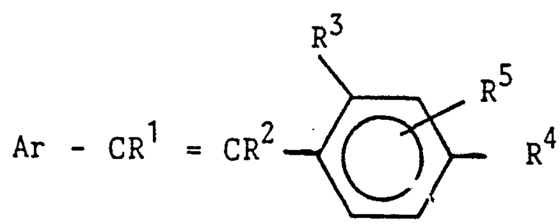

(I)

worin

$R^1$ und $R^2$ Wasserstoffatome oder Methylgruppen bedeuten,

$R^3$ und $R^4$ voneinander verschieden sind und jeweils ein Wasserstoffatom oder einen 4,6-Bis-trichlormethyl-s-triazin-2-ylrest bedeuten,

$R^5$, ein Wasserstoff- oder Halogenatom, einen Alkyl-, oder Alkoxyrest und

Ar Phenyl bedeutet, das 1- bis 3-fach durch Halogenatome, Alkyl-, Halogenalkyl-, Aralkyl-, Aryloxyalkyl-, Cycloalkyl-, Alkenyl-, Aryl- oder Aryloxyreste substituiert sein kann, wobei in den Alkylresten einzelne Methylengruppen durch O- oder S-Atome ersetzt, jeweils zwei dieser Reste unter Ausbildung eines 5- oder 6-gliedrigen Rings verknüpft sein können und die Substituenten insgesamt nicht mehr als 12 Kohlenstoffatome aufweisen, oder aber ein Naphthyl-, Acenaphthyl-, Di- oder Tetrahydronaphthyl-, Indanyl-, Anthryl-, Phenanthryl-, Fluorenyl- oder Tetrahydrophenanthrylrest ist, der durch Halogenatome, Alkylreste mit 1 bis 3, Alkoxyreste mit 1 bis 4 oder Alkoxyalkylreste mit 3 bis 6 Kohlenstoffatomen substituiert sein kann.

Erfindungsgemäß wird weiterhin ein lichtempfindliches Gemisch vorgeschlagen, das ein Bis-trichlormethyl-s-triazin (a) und eine Verbindung (b) enthält, die mit dem Lichtreaktionsprodukt des Triazins (a) unter Ausbildung eines Produkts zu reagieren vermag das eine von (b) unterschiedliche Lichtabsorption oder Löslichkeit in einem Entwickler aufweist. Das erfindungsgemäße Gemisch ist dadurch gekennzeichnet, daß das Triazin (a) eine Verbindung gemäß der oben definierten Formel I ist.

Die erfindungsgemäßen Verbindungen bilden unter Einwirkung von aktinischer Strahlung freie Radikale, die zur Einleitung chemischer Reaktionen, insbesondere von radikalisch initiierten Polymerisationen, befähigt sind. Sie bilden ferner bei Bestrahlung Halogenwasserstoff, durch den säurekatalysierte Reaktionen, z. B. die Spaltung von Acetalbindungen, oder Salzbildungen, z. B. Farbumschläge von Indikatorfarbstoffen, in Gang gesetzt werden können.

Als aktinische Strahlung soll im Rahmen dieser Beschreibung jede Strahlung verstanden werden, deren Energie mindestens der des kurzwelligen sichtbaren Lichts entspricht. Geeignet ist insbesondere langwellige UV-Strahlung, aber auch Elektronen-, Röntgen- oder Laserstrahlung.

In der oben angegebenen allgemeinen Formel I haben die Symbole vorzugsweise die folgende Bedeutung:

$R^1$ und $R^2$ sind Wasserstoffatome,

$R^5$ ist ein Wasserstoff-, Chlor- oder Bromatom, ein Alkylrest mit 1 bis 3 C-Atomen, oder eine Methoxygruppe,

Ar ist ein Phenylrest der Formel II

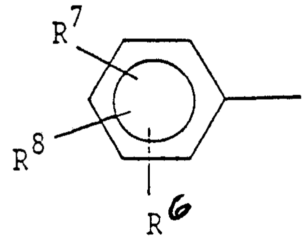

(II)

worin

$R^6$ bis $R^8$ gleich oder verschieden sind und Wasserstoffatome oder die vorstehend angegebenen Substituenten bedeuten.

Besonders bevorzugt sind Verbindungen der Formel I, worin

$R^1$, $R^2$, $R^3$ und $R^5$ Wasserstoffatome bedeuten und

Ar der Formel II entspricht, in welcher

$R^6$ bis $R^8$ gleich oder verschieden sind und Wasserstoff-, Fluor-, Chlor- oder Bromatome, Alkyl-, Alkoxy- oder Alkoxyalkylreste bedeuten

oder

$R^6$ ein Wasserstoffatom und

$R^7$ und $R^8$ gemeinsam eine Dioxymethylengruppe bedeuten.

Eine ganz besonders bevorzugte Verbindung ist 2-(4-Styrylphenyl)-4,6-bis-trichlormethyl-s-triazin.

Im einzelnen kann unter den besonders bevorzugten Verbindungen der Rest Ar z. B. die folgende Bedeutung haben: Phenyl, 2-, 3- oder 4-Fluorphenyl, 2-, 3- oder 4-Chlorphenyl, 2-, 3- oder 4-Bromphenyl, 2-, 3- oder 4-Methyl-, -Ethyl-, -Propyl-, -Butyl-, -Isobutyl-, -Hexyl-, -Nonyl- oder -Dodecylphenyl, 2-, 3- oder 4-Methoxy-, -Ethoxy-, -Isopropoxy-, -Butoxy-, -Pentoxy-, -Octyloxy- oder -Decyloxyphenyl, 2,4-Dichlor- oder -Dibromphenyl, 3,4-Dichlor- oder -Dibromphenyl, 2,6-Dichlorphenyl, 3-Brom-4-fluorphenyl, 2,3-, 2,4-, 2,5-, 3,4- oder 3,5-Dimethoxy-, -Diethoxy, -Dibutoxy- oder -Dihexoxyphenyl, 2-Ethoxy-5-methoxyphenyl, 3-Chlor-4-Methylphenyl, 2,4-Dimethylphenyl, 2-, 3- oder 4-Methoxyethyl-, -Ethoxyethyl- oder -Butoxyethylphenyl, 2,4,6-Trimethylphenyl, 3,4,5-Trimethoxy- oder -Triethoxyphenyl, 2,3-Dioxymethylen-phenyl oder 3,4-Dioxymethylen-phenyl.

Die Aryl-bis-trichlormethyl-s-triazine der Erfindung werden in einfachster und bevorzugter Weise durch Co-trimerisation von 1 mol Arylcarbonsäurenitril der Formel III:

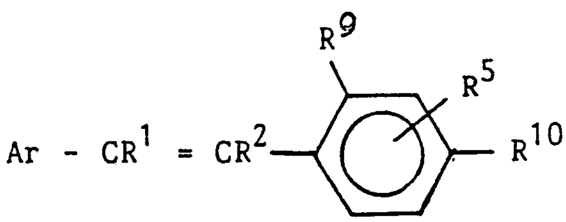

(III)

worin

$R^9$ und $R^{10}$ voneinander verschieden sind und jeweils ein Wasserstoffatom oder einen CN-Rest bedeuten und

Ar, $R^1$, $R^2$ und $R^5$ die im Anspruch 1 angegebene Bedeutung haben,

und 2 - 8 mol Trichloracetonitril in Gegenwart von Halogen-, vorzugsweise Chlorwasserstoff und Friedel-Crafts-Katalysatoren wie $AlCl_3$, $AlBr_3$, $TiCl_4$ und Bortrifluoridetherat analog dem in Bull. Chem. Soc. Jap. 42, 2924 (1969) beschriebenen Vorgehen hergestellt. Andere Synthesemöglichkeiten bestehen in der Umsetzung der Arylamidine mit Polychlor-3-aza-pent-3-en entsprechend der in Angew. Chem. 78, 982 (1966) veröffentlichten Methode oder beispielsweise in der Reaktion von Carbonsäurechloriden oder -anhydriden mit N-(Iminoacyl)-trichloracetamidinen, durch die auch 2-Aryl-4-methyl-6-trichlormethyl-s-triazine glatt zugänglich sind, wie es in der GB-A-912 112 beschrieben ist.

Die zur Co-trimerisation eingesetzten Nitrile können besonders einfach synthetisiert werden durch Horner-Wittig-Olefinierung (s. "Houben-Weyl", Bd. 5/1b, S. 396 - 401 und 895 - 899) nach dem Schema:

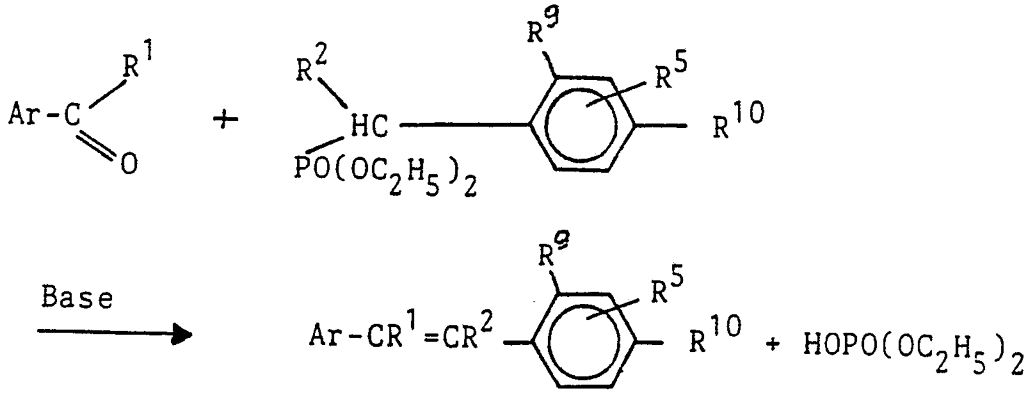


wobei $R^9$ oder $R^{10}$ den Nitrilrest kennzeichnen und alle anderen Reste die unter der Formel I beschriebene Bedeutung haben. Der Aralkylphosphonsäurediethylester entsteht glatt durch Umsetzung des entsprechenden α-Halogenalkylaromaten mit Triethylphosphit.

Selbstverständlich können die Nitrile auch nach anderen literaturbekannten Methoden durch Austausch oder aus den entsprechenden Carbonsäuren oder Carbonsäurederivaten hergestellt werden. In "Methoden der Organ. Chemie" (Houben-Weyl), Bd. 5/1b (1972) finden sich eine Fülle von Synthesen substituierter Stilbene.

Die erfindungsgemäßen neuen Verbindungen sind als Photoinitiatoren für photopolymerisierbare Schichten geeignet, die als wesentliche Bestandteile Monomere, Bindemittel und Initiatoren enthalten.

Für diese Anwendung brauchbare photopolymerisierbare-Monomere sind bekannt und z. B. in den US-A-2 760 863 und 3 030 023 beschrieben.

Bevorzugte Beispiele sind Acryl- und Methacrylsäureester mehrwertiger Alkohole, wie Diglycerindiacrylat, Polyethylenglykoldimethacrylat, Acrylate und Methacrylate von Trimethylolethan, Trimethylolpropan und Pentaerythrit und von mehrwertigen alicyclischen Alkoholen. Mit Vorteil werden auch Umsetzungsprodukte von Diisocyanaten mit Partialestern mehrwertiger Alkohole eingesetzt. Derartige Monomere sind in den DE-A-2 064 079, 2 361 041 und 2 822 190 beschrieben.

Der Mengenanteil der Schicht an Monomeren beträgt im allgemeinen 10 bis 80, vorzugsweise 20 bis 60 Gew.-%.

Als Bindemittel können eine Vielzahl löslicher organischer Polymerisate Einsatz finden. Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrig-alkalischen Entwicklern entwickeln lassen. Derartige Bindemittel können z. B. die folgenden Gruppen enthalten:

$-COOH$, $-PO_3H_2$, $-SO_3H$; $-SO_2NH-$, $-SO_2NHSO_2-$ und $-SO_2-NH-CO-$.

Als Beispiele hierfür seien genannt: Maleinatharze, Polymerisate aus N-(p-Tolyl-sulfonyl)-carbaminsäure-(β-methacryloyloxy-ethyl)ester und Mischpolymerisate dieser und ähnlicher Monomerer mit anderen Monomeren sowie Styrol-Maleinsäureanhydrid-Mischpolymerisate. Alkylmethacrylat-Methacrylsäure-Mischpolymerisate und Mischpolymerisate aus Methacrylsäure, Alkylmethacrylaten und Methylmethacrylat und/oder Styrol, Acrylnitril u. a., wie sie in den DE-A-2 064 080 und 2 363 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:

Inhibitoren zur Verhinderung der thermischen Polymerisation der Monomeren, Wasserstoffdonatoren, die Abbildungseigenschaften derartiger Schichten modifizierende Stoffe, Farbstoffe, gefärbte und ungefärbte Pigmente, Farbbildner, Indikatoren oder Weichmacher.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Kopiermaterial auf dem Reproduktionsgebiet. Als Anwendungsmöglichkeiten auf diesem Gebiet seien genannt: Kopierschichten für die photomechanische Herstellung von Druckformen für den Hochdruck, den Flachdruck, den Tiefdruck, den Siebdruck, von Reliefkopien, z. B. Herstellung von Texten in Blindenschrift, von

Einzelkopien, Gerbbildern, oder Pigmentbildern. Weiter sind die Gemische zur photomechanischen Herstellung von Ätzreservagen, z. B. für die Fertigung von Namensschildern, von kopierten Schaltungen und für das Formteilätzen, anwendbar.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung gedruckter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen. Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Wachse, Polyvinylalkohol, Polyphosphate, Zucker usw.

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Die strahlungsempfindlichen Verbindungen sind als Photoinitiatoren bereits in Konzentrationen von etwa 0,05 % des Gesamtfeststoffs der Masse wirksam, eine Erhöhung über 10 % ist im allgemeinen unzweckmäßig. Vorzugsweise werden Konzentrationen von 0,3 bis 7 % verwendet.

Weiterhin können die erfindungsgemäßen Verbindungen auch in solchen strahlungsempfindlichen Gemischen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Reaktionen auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säuren erfolgt, deren Anionen eine geringere Nukleophilie als Chlorid besitzen, also als das Anion der bei der Photolyse der neuen Verbindungen entstehenden Säuren, so härten doch Schichten, die aus Epoxyharzen und Novolaken bestehen, bei Belichtung in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farbvorläufern, z. B. Leukoverbindungen, Farbbildung zu induzieren oder bathochrome Farbverschiebungen und -vertiefungen in Gemischen zu bewirken, die Cyanin-, Merocyanin- oder Styrylfarbbasen enthalten. Auch kann z. B. in den in der DE-A-1 572 080 beschriebenen Gemischen, die Farbbase, N-Vinylcarbazol und einen Halogenkohlenwasserstoff enthalten, die Halogenverbindung Tetrabrommethan durch einen Bruchteil, d. h. etwa 2 %, ihrer Menge an erfindungsgemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können. Statt der in den DE-A-2 331 377 und 2 641 100 genannten Säurespender sind vorteilhaft die vorliegenden Verbindungen zu benutzen.

Ein besonders bevorzugtes Anwendungsgebiet für die erfindungsgemäßen Verbindungen sind Gemische, die neben ihnen als wesentliche Komponente eine Verbindung mit mindestens einer durch Säure spaltbaren C-O-C-Gruppierung enthalten. Als durch Säure spaltbare Verbindungen sind in erster Linie zu nennen:

A) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können und

B) Polymerverbindungen mit wiederkehrenden Acetal- und/oder Ketalgruppierungen, bei denen vorzugsweise beide α-C-Atome der zum Aufbau dieser Gruppierungen erforderlichen Alkohole aliphatisch sind.

Durch Säure spaltbare Verbindungen des Typs A als Komponenten strahlungsempfindlicher Gemische sind in den DE-A-2 610 842 und 2 928 636 ausführlich beschrieben; Gemische, die Verbindungen des Typs B enthalten, sind Gegenstand der DE-C-2 718 254.

Als durch Säure spaltbare Verbindungen sind z. B. auch die speziellen Aryl-alkyl-acetale und -aminale der DE-C-2 306 248 zu nennen, die durch die Photolyseprodukte der erfindungsgemäßen Verbindungen ebenfalls abgebaut werden. Weitere Verbindungen sind Enolether und Acyliminocarbonate, die in den EP-A-6 626 und 6 627 beschrieben sind.

Solche Gemische, in denen durch Einwirkung von aktinischer Strahlung mittelbar oder unmittelbar Moleküle, deren Anwesenheit die chemischen und/oder physikalischen Eigenschaften des Gemischs wesentlich beeinflußt, in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden. Bei Kopiermaterialien spricht man in diesen Fällen von positiv arbeitenden Systemen.

Die bei vielen Positiv-Kopiermaterialien bewährten Novolak-Kondensationsharze haben sich als Zusatz auch

bei der Anwendung der erfindungsgemäßen Verbindungen in Gemischen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55 - 85 Gew.-%. Statt oder im Gemisch mit Novolaken sind vorteilhaft auch andere Phenolgruppen tragende Harze verwendbar. Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann das lichtempfindliche Gemisch für spezielle Erfordernisse wie Flexibilität, Haftung und Glanz etc. außerdem noch Substanzen wie Polyglykole, Cellulose-Derivate wie Ethylcellulose, Netzmittel, Farbstoffe und feinteilige Pigmente sowie bei Bedarf UV-Absorber enthalten.

Entwickelt wird vorzugsweise mit in der-Technik üblichen wäßrig-alkalischen Entwicklern, die auch kleine Anteile organischer Lösemittel enthalten können. Photopolymerisierbare Gemische können auch mit organischen Lösemitteln entwickelt werden.

Die bereits im Zusammenhang mit den photopolymerisierbaren Gemischen aufgeführten Träger kommen ebenfalls für positiv arbeitende Kopiermaterialien in Frage, zusätzlich die in der Mikroelektronik üblichen Silizium-, Siliziumnitrid- und Siliziumdioxid-, Metall- und Polymeroberflächen.

Die Menge der als Photoinitiator eingesetzten erfindungsgemäßen Verbindungen kann in den positiv arbeitenden Gemischen je nach Substanz und Schicht sehr verschieden sein. Günstigere Ergebnisse werden erhalten zwischen 0,05 und 12 %, bezogen auf den Gesamtfeststoff, bevorzugt sind 0,1 bis 8 %. Für Schichten mit Dicken über 10 µm empfiehlt es sich, relativ wenig Säurespender zu verwenden.

Grundsätzlich ist elektromagnetische Strahlung mit Wellenlängen bis etwa 550 nm zur Belichtung geeignet. Der bevorzugte Wellenlängenbereich erstreckt sich von 220 bis 500 nm.

Die Vielfalt der erfindungsgemäßen Verbindungen, deren Absorptionsmaxima teilweise noch weit im sichtbaren Teil des Spektrums zu finden sind und deren Absorptionsbereich über 500 nm hinausreicht, gestattet es einerseits, den Photoinitiator optimal auf die verwendete Lichtquelle abzustimmen. Prinzipiell ist auch eine Sensibilisierung nicht ausgeschlossen. Andererseits ist es aber auch möglich und im Sinne der Erfindung zweckmäßig, denselben Photoinitiator in strahlungsempfindlichen Massen zu verwenden, die Strahlung unterschiedlicher Strahlungsquellen und damit Strahlung verschiedener Wellenlänge ausgesetzt werden. Eine große Zahl der erfindungsgemäßen Photoinitiatoren zeigt hervorragende Ergebnisse sowohl in strahlungsempfindlichen Massen für die Verarbeitung in mit Argon-Ionen-Lasern bestückten automatischen Belichtungsgeräten wie in Kopiermaterialien, die mit metallhalogenid-dotierten Quecksilber-Hochdrucklampen belichtet werden. Als weitere Lichtquellen sind beispielsweise zu nennen:

Röhrenlampen, Xenonimpulslampen und Kohlebogenlampen. Darüber hinaus ist bei den erfindungsgemäßen lichtempfindlichen Gemischen das Belichten in üblichen Projektions- und Vergrößerungs-Geräten unter dem Licht der Metallfadenlampen und Kontakt-Belichtung mit gewöhnlichen Glühbirnen möglich. Die Belichtung kann auch mit anderen Lasern erfolgen. Geeignet für die Zwecke vorliegender Erfindung sind leistungsgerechte kurzwellige Laser, beispielsweise Excimer-Laser, Krypton-Ionen-Laser, Farbstoff-Laser und Helium-Cadmium-Laser, die insbesondere zwischen 190 und 550 nm emittieren.

Das Bestrahlen mit Elektronenstrahlen ist eine weitere Differenzierungsmöglichkeit. Elektronenstrahlen können Gemische, die eine der erfindungsgemäßen Verbindungen und eine durch Säure spaltbare Verbindung enthalten, wie auch viele andere organische Materialien durchgreifend zersetzen und vernetzen, so daß ein negatives Bild entsteht, wenn die unbestrahlten Teile durch Lösemittel oder Belichten ohne Vorlage und Entwickeln entfernt werden. Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die günstigsten Bedingungen können durch Vorversuche leicht ermittelt werden.

Bevorzugte Anwendung finden die eine der erfindungsgemäßen Verbindungen enthaltenden strahlungsempfindlichen Gemische bei der Herstellung von Druckformen, d. h. insbesondere Offset-, autotypischen Tiefdruck- und Siebdruckformen, in Photoresistlösungen und in sogenannten Trockenresists.

Sie lassen sich auch vorteilhaft bei der Herstellung von Klebefolien entsprechend WO-A 81/02261 anstelle der dort genannten Naphthyl-bis-trichlormethyl-s-triazine einsetzen.

Die folgenden Beispiele dienen zur näheren Erläuterung der Erfindung. In den Beispielen stehen Gew.-Teile (Gt) und Vol.-Teile (Vt) im Verhältnis von g zu ml. Prozent- und Mengenangaben sind, wenn nichts anderes angegeben ist, in Gewichtseinheiten zu verstehen.

Es wird zunächst die Herstellung einer Anzahl der neuen Arylvinylphenyl-bis-trichlormethyl-s-triazine beschrieben, die in erfindungsgemäßen lichtempfindlichen Gemischen als säureabspaltende und radikalerzeugende Verbindungen erprobt wurden. Sie wurden als Verbindung 1 bis 19 durchnumeriert und kehren unter dieser Bezeichnung in den Anwendungsbeispielen wieder. Die als Ausgangsverbindungen eingesetzten Arylvinylphenylcarbonitrile der Formel III sind z. T. literaturbekannt, teils wurden sie analog der für den einfachsten Vertreter wiedergegebenen Methode hergestellt.

Auch für die Herstellung der als Ausgangsverbindungen für die Verbindungen Nr. 2 - 12, 18 und 19 dienenden

Nitrile wurde 4-Cyano-phenylmethanphosphonsäurediethylester als P-O-aktivierte Komponente mit dem Aldehyd Ar-CHO entsprechend der Nomenklatur der Tabelle 1 umgesetzt; zur Herstellung des Nitrils der Verbindung 16 wird mit Acetophenon umgesetzt. P-O-aktivierte Komponente bei der Herstellung des Ausgangsnitrils für die Verbindung 17 war 2-Cyano-phenylmethanphosphonsäurediethylester. 2-Chlor-4-cyano-phenylmethanphosphonsäurediethylester diente zur Synthese der Ausgangsnitrile für die Verbindungen 13 bis 15. Der Phosphonsäureester wurde auf folgendem Wege erhalten: 3-Chlor-4-methyl-benzoesäure wurde über das Säurechlorid und das Amid durch Dehydratisierung mit Thionylchlorid in das 3-Chlor-4-methyl-benzonitril übergeführt, dieses mit N-Bromsuccinimid in Tetrachlormethan in das 4-Brommethyl-3-chlor-benzonitril umgewandelt und daraus durch Umsetzung mit Triethylphosphit der Phosphonsäureester erhalten.

**Allgemeine Vorschrift zur Herstellung der Arylvinylphenylcarbonitrile anhand der Darstellung von Stilben-4-carbonitril**

In die kräftig gerührte Mischung von 22 g gepulvertem Kaliumhydroxid und 200 ml Dimethylformamid tropft man unter Eiskühlung in 2 Stunden eine Lösung von 0,1 mol Benzaldehyd in 0,105 mol 4-Cyano-phenylmethanphosphonsäurediethylester. Man rührt unter fortgesetzter Kühlung 1 Stunde nach und eine weitere Stunde ohne Kühlung. Dann gießt man das Gemisch in 1 l Eiswasser, das 52 ml konz. Salzsäure enthält. Der Niederschlag wird abgesaugt, mit Wasser chloridionenfrei gewaschen, getrocknet und aus Methanol umkristallisiert.

Stilben-4-carbonitril vom Schmp. 116 - 118° C wird in einer Ausbeute von 84 % d. Th. erhalten.

**Allgemeine Vorschrift zur Herstellung der 2-(4-Arylvinylphenyl)-4,6-bis-trichlormethyl-s-triazine anhand der Herstellung von 2-(4-Styrylphenyl)-4,6-bis-trichlormethyl-s-triazin**

In eine gerührte Suspension aus 0,5 mol Stilben-4-carbonitril, 3,0 mol Trichloracetonitril und 0,06 mol Aluminiumtribromid wird bei 24 - 28° C Chlorwasserstoffgas bis zur Sättigung eingeleitet.

Dabei lösen sich die Feststoffe fast vollständig. Danach bildet sich ein Niederschlag, der Kolbeninhalt nimmt breiige Konsistenz an, und Chlorwasserstoffabspaltung setzt ein. Durch Kühlung wird das Gemisch 6 Stunden auf 28 - 30° C gehalten, dann 24 - 48 Stunden bei Raumtemperatur stehen gelassen. Das Reaktionsgemisch wird in 1,6 l Methylenchlorid aufgenommen, die Lösung mit Wasser neutral gewaschen und über Natriumsulfat getrocknet. Nach Abdestillieren des Lösungsmittels unter vermindertem Druck nimmt man den Rückstand in 600 ml Methylenchlorid auf und versetzt die Lösung mit 1700 ml Methanol. 210 g (85 % d. Th.) noch leicht verunreinigtes 2-(4-Styrylphenyl)-4,6-bis-trichlormethyl-s-triazin kristallisieren aus, das noch durch weiteres Umkristallisieren aus Methylenchlorid/Methanol gereinigt werden kann. Doppelschmp. 160 - 163 und 170 - 173° C.

Als Katalysator eignet sich auch $AlCl_3$. Bortrifluoridetherat ist ein weniger wirksamer Katalysator, ergibt aber sehr reine Reaktionsprodukte.

Entsprechend werden die in der Tabelle 1 aufgeführten Triazine dargestellt, wobei in einigen Fällen eine chromatographische Reinigung der Endprodukte erfolgt.

**Tabelle 1** Bis-trichlormethyl-s-triazine der allgemeinen Formel I mit $R^2$ = H; $R^5$ = H;

| Verbindung Nr. | Ar | $R^1$ | $R^3$ | $R^4$ | $R^6$ | $R^7$ | $R^8$ | λ (EtOH)/log ε | Schmp (°C) |
|---|---|---|---|---|---|---|---|---|---|
| 1 | Formel II | H | H | X | H | H | H | 371 nm/4.62 | 160 - 163 und 170 - 173 |
| 2 | " | H | H | X | H | H | 4-$CH_3O$ | 395 nm/4.60 | 203 - 207 |
| 3 | " | H | H | X | H | H | 4-$CH_3$ | 381 nm/4.61 | 188 - 192 |
| 4 | " | H | H | X | H | H | 4-Cl | 371 nm/4.65 | 192 - 194 |
| 5 | " | H | H | X | 2-Cl | H | H | 362 nm/4.62 | 208 - 210 |
| 6 | " | H | H | X | H | 3-Cl | H | 365 nm/4.64 | 193 - 196 |
| 7 | " | H | H | X | H | H | 4-Br | 371 nm/4.65 | 210 - 211 |
| 8 | " | H | H | X | H | 3-$CH_3O$ | 4-$CH_3O$ | 400 nm/4.57 | 126 - 129 |
| 9 | " | H | H | X | H | 3,4-$OCH_2O$ | | 398 nm/4.57 | 180 - 184 |
| 10 | " | H | H | X | H | H | 4-$C_2H_5O$ | 397 nm/4.60 | 189 - 193 |
| 11 | " | H | H | X | 5-$CH_3O$ | 3-$CH_3O$ | 4-$CH_3O$ | 389 nm/4.58 | 209 - 214 |
| 12 | " | H | H | X | H | H | 4-n-$C_6H_{13}$ | 382 nm/4.51 | 75 - 77 |
| 13 | " | H | Cl | X | H | H | H | 369 nm/4.56 | 215 - 219 |
| 14 | " | H | Cl | X | H | 3-Cl | H | 362 nm/4.58 | 191 - 194 |
| 15 | " | H | Cl | X | H | H | 4-$CH_3$ | 379 nm/4.56 | 210 - 213 |
| 16 | " | $CH_3$ | H | X | H | H | H | 355 nm/4.35 | 169 - 172 |

| | | | | | | | | | |
|---|---|---|---|---|---|---|---|---|---|
| 17 | " | H | X | H | H | H | H | 372 nm/3.80 | 107 - 111 |
| 18 | Naphth-1-yl | H | H | X | | | | 385 nm/4.51 | 210 - 214 |
| 19 | Anthrac-9-yl | H | H | X | | | | 408 nm/4.20 | 160 - 172 |

X = 4,6-Bis-trichlormethyl-s-triazin-2-yl

**Beispiel 1**

Aluminiumplatten mit elektrochemisch aufgerauhter, anodisierter und mit einer 0,1-%-igen wäßrigen Lösung von Polyvinylphosphonsäure vorbehandelter Oberfläche werden mit Lösungen von

6,63 Gt eines Kresol-Formaldehyd-Novolaks (Schmelzbereich 105 - 120°C nach DIN 53 181),
1,99 Gt des polymeren Orthoesters, hergestellt durch Kondensation von Orthoameisensäuretrimethylester mit 4-Oxa-6,6-bis-hydroxymethyl-octan-1-ol,
0,33 Gt der Verbindung Nr. 1 und
0,05 Gt Kristallviolett-Base in
30 Gt Ethylenglykolmonomethylether
52 Gt Tetrahydrofuran und
9 Gt Butylacetat

beschichtet, so daß nach dem Trocknen eine Schichtdicke von ca. 2,0 µm resultiert. Die lichtempfindliche Schicht wird durch eine Vorlage, die neben Strich- und Rastermotiven einen Halbtonstufenkeil mit 13 Stufen der optischen Dichte von je 0,15 enthält, unter einer 5 kW-Metallhalogenidlampe im Abstand von 110 cm 15 Sekunden belichtet und nach einer Wartezeit von 10 Minuten mit dem Entwickler der folgenden Zusammensetzung 1 Minute entwickelt:

5,5 Gt Natriummetasilikat x 9 $H_2O$,
3,4 Gt Trinatriumphosphat x 12 $H_2O$,
0,4 Gt Natriumdihydrogenphosphat, wasserfrei,
90,7 Gt Wasser.

Man erhält ein positives Abbild der Vorlage. Ein Druckversuch mit der so hergestellten Druckform in einer Offsetdruckmaschine wird nach 140 000 Drucken bei immer noch einwandfreier Qualität abgebrochen.

Das in diesem Beispiel erzielte Kopierergebnis, gemessen an der Zahl der entwickelten Halbtonstufen, wird ebenfalls erreicht, wenn die Verbindung Nr. 1 durch jeweils gleiche Gewichtsmengen einer der Verbindungen Nr. 3, 4, 5, 6, 7, 12 und 13 bzw. von Gemischen dieser Verbindungen ersetzt wird.

Versieht man die in der DE-C-2 718 259 als besonders bevorzugt hervorgehobenen Initiatoren Nr. 5 und Nr. 20 2-(4-Ethoxynaphth-1-yl)- bzw. 2-Acenaphth-5-yl-4,6-bis-trichlormethyl-s-triazin mit den Lichtempfindlichkeitsfaktoren, 100 bzw. 125, so sind den obengenannten Photoinitiatoren Faktoren zwischen 135 und 140 zuzuordnen.

**Beispiel 2**

Druckplatten des Formats 580 mm x 420 mm, die aus einer elektrochemisch aufgerauhten, anodisierten und mit Polyvinylphosphonsäure vorbehandelten Aluminiumfolie und einer lichtempfindlichen Schicht der folgenden Zusammensetzung:

73,96 Gt des Novolaks nach Beispiel 1,
22,19 Gt des Polyorthoesters nach Beispiel 1,
3,70 Gt Photoinitiator und
0,15 Gt Kristallviolett-Base

bestehen, werden in einer Laserite®-Anlage mit der UV-Strahlung eines Argon-Ionen-Lasers zeilenweise belichtet, wobei die Zahl der geschriebenen Zeilen/cm stufenweise gesteigert wird. Die Leistung des Lasers wird bei diesem Vorgehen konstant gehalten. Die belichtete Druckplatte wird 10 Minuten bei Raumtemperatur gelagert, anschließend mit dem Entwickler des Beispiels 1 60 Sekunden entwickelt und mit fetter Farbe eingefärbt. Ab einer bestimmten Zeilenzahl/cm sind die Nichtbildstellen klar und nehmen keine Farbe mehr an.

Aus der Laserleistung an der Plattenoberfläche, der Zeilenlänge, der Zeilenzahl/sec und der ermittelten Zeilenzahl/cm läßt sich der vom verwendeten Photoinitiator abhängige Mindestenergiebedarf errechnen, der für eine tonfreie Entwickelbarkeit der Nichtbildstellen erforderlich ist. Die folgende Tabelle nennt diese Energiewerte:

| Verbindung | Mindestenergiebedarf (mJ/cm²) |
|---|---|
| 1 | 6,5 |
| 3 | 7,5 |
| 4 | 6,7 |
| 5 | 5,6 |
| 6 | 6,5 |
| 7 | 6,5 |
| 12 | 7,8 |
| 13 | 5,6 |
| 14 | 7,2 |
| 16 | 7,3 |

Zum Vergleich:

| | |
|---|---|
| 2-(4-Ethoxynaphth-1-yl)-4,6-bis-trichlormethyl-s-triazin | 8,4 |
| 2-Acenaphth-5-yl-4,6-bis-trichlormethyl-s-triazin | 11,2 |
| 2-(4-Methoxystyryl)-4,6-bis-trichlormethyl-s-triazin | 9,2 |

Der Vergleich zeigt, daß der gegenüber der Emission von Metallhalogenidlampen hochaktive, jedoch von den neuen Photoinitiatoren noch übertroffene Photoinitiator 2-Acenaphth-5-yl-4,6-bis-trichlormethyl-s-triazin einen um 100 % höheren Mindestenergiebedarf bei Laserbelichtung gegenüber den Verbindungen Nr. 5 und Nr. 13 aufweist.

**Beispiel 3**

Eine auf mechanisch aufgerauhte Aluminiumfolie aus 10-%-iger Methylethylketon-Lösung aufgetragene Schicht der folgenden Zusammensetzung

76,63 Gt Phenol-Formaldehyd-Novolak (Schmelzbereich 110 - 120°C nach DIN 53 181),
19,16 Gt des Polyacetals aus 2-Ethylbutyraldehyd und Triethylenglykol,
3,83 Gt der Verbindung Nr. 18 und
0,38 Gt Kristallviolett-Base

wird 5 Sekunden unter den Bedingungen des Beispiels 1 belichtet und 45 Sekunden mit dem Entwickler des Beispiels 1 entwickelt. Man erhält eine Druckform mit dem positiven Abbild der Vorlage.

Eine etwas verringerte Empfindlichkeit wird mit der Verbindung Nr. 19 erzielt.

**Beispiel 4**

Die Eignung der neuen Bis-trichlormethyl-s-triazine als Initiatoren in elektronenstrahlempfindlichen Massen wird im folgenden gezeigt:

Auf mechanisch aufgerauhtes Aluminium ca. 1,1 µm dick aufgetragene Schichten der Zusammensetzung

73 Gt des Novolaks nach Beispiel 1,
22 Gt des Bis-(5-butyl-5-ethyl-1,3-dioxan-2-yl)-ethers des 2-Butyl-2-ethyl-propandiols und
5 Gt einer der Verbindungen Nr. 2, Nr. 9 und Nr. 10

werden mit 11 kV-Elektronen bestrahlt.

Bei einem Strahlstrom von 5 µA reichen 4 Sekunden Bestrahlungsdauer aus, um ein Feld von 10 cm² nach 60 Sekunden Einwirkung des Entwicklers aus Beispiel 1 löslich zu machen; das entspricht einer Empfindlichkeit der obengenannten Schichten von 2 µC/cm².

**Beispiel 5**

Eine Platte aus elektrolytisch aufgerauhtem und anodisiertem Aluminium wird mit einer Beschichtungslösung, bestehend aus

6,7 Gt Trimethylolethan-triacrylat,
6,5 Gt eines Copolymerisats aus Methylmethacrylat und Methacrylsäure, Säurezahl 115,
0,12 Gt der Verbindung Nr. 1,
64,0 Gt Ethylenglykolmonoethylether,
22,7 Gt Butylacetat und
0,3 Gt 2,4-Dinitro-6-chlor-2'-acetamido-5'-methoxy-4'-(N-β-hydroxyethyl-N-β'-cyanoethyl-amino)-azobenzol

schleuderbeschichtet, so daß sich nach dem Trocknen ein Schichtgewicht von 3 - 4 g/m$^2$ ergibt. Anschließend wird die Platte mit einer 4 µm dicken Schutzschicht aus Polyvinylalkohol (K-Wert 4; 12 % Restacetylgruppen) versehen, unter einer Strich- und Rastervorlage mit einer 5 kW-Metallhalogenidlampe im Abstand von 110 cm 22 Sekunden belichtet und mit einer 1,5-%-igen Natriummetasilikatlösung entwickelt.

Man erhält ein negatives Abbild der Vorlage. Ein Druckversuch mit einer so hergestellten Offsetdruckplatte wurde nach 200 000 Drucken bei immer noch einwandfreier Qualität abgebrochen.

**Beispiel 6**

Dieses Beispiel beschreibt einen Negativ-Trockenresist. Die folgende Beschichtungslösung aus

24,9 Gt eines Mischpolymerisats aus 30 Gt Methacrylsäure, 60 Gt n-Hexylmethacrylat und 10 Gt Styrol,
16,1 Gt des Umsetzungsprodukts aus 1 mol 2,2,4-Trimethylhexamethylen-diisocyanat und 2 mol Hydroxyethylmethacrylat,
0,41 Gt Triethylenglykoldimethacrylat,
0,58 Gt der Verbindung Nr. 5
0,11 Gt des in Beispiel 5 angegebenen Farbstoffs und
57,9 Gt Methylethylketon

wird auf eine Polyethylenterephthalatfolie zu einem Trockengewicht von 25 g/m$^2$ aufgeschleudert. Dieses Material wird in einem handelsüblichen Laminator bei 120°C auf einen Träger aus Isolierstoffmaterial, der eine 35 µm dicke Kupferauflage trägt, laminiert. Nach 60 Sekunden Belichtung durch eine Vorlage, die neben Strich- und Rastermotiven einen Halbtonstufenkeil enthält, unter der Lichtquelle aus Beispiel 1 und Entwicklung mit 0,8-%-iger Natriumcarbonatlösung bleiben ein Negativ des Strich- und Rastermotivs sowie die Stufen 1 - 6 des Halbtonkeils als erhabenes Relief stehen, die Stufe 7 ist teilweise angegriffen.

Die Resistschicht widersteht Ätzprozessen, z. B. mit Eisen-III-chlorid, und den Einflüssen von Galvanobädern bei der Herstellung von Leiterplatten.

**Beispiel 7**

Auf einer Schleuder wird eine Platte aus mechanisch aufgerauhtem Aluminium mit einer Lösung aus

4,3 Gt eines Phenol-Formaldehyd-Novolaks (Schmelzbereich 110 - 120°C nach DIN 53 181),
10,6 Gt N-Vinylcarbazol,
0,24 Gt 2-(p-Dimethylaminostyryl)-benzthiazol,
0,25 Gt einer der Verbindungen Nr. 15 und Nr. 18,
84,6 Gt Methylethylketon

beschichtet. Nach dem Trocknen hat die lichtempfindliche Schicht eine Dicke von 1 - 2 µm. Die Platte wird 7,5 Sekunden entsprechend Beispiel 1 bildmäßig belichtet, wobei an den Bildstellen der Farbton der Schicht von gelb nach rot-orange umschlägt. Schaukeln der Platte in der Entwicklerlösung aus

0,6 Gt NaOH,
0,5 Gt $Na_2SiO_3 \cdot 5\ H_2O$,
1,0 Gt n-Butanol und
97,9 Gt vollentsalztem Wasser

entfernt in 75 Sekunden die unbelichteten Schichtanteile, die belichteten nehmen beim Überwischen fette Farbe an, so daß von einer solchen Platte auf einer Offsetdruckmaschine gedruckt werden kann.

Bei etwas verlängerten Belichtungszeiten lassen sich auch die Verbindungen Nr. 8, 11 oder 17 anstelle der Verbindungen Nr. 15 und Nr. 18 verwenden.

**Beispiel 8**

Das Beispiel 7 wird wiederholt, indem in der Beschichtungslösung die Styrylfarbbase durch die gleiche Gewichtsmenge der Cyaninfarbbase 2-[1-Cyano-3-(3-ethylbenzthiazolyliden-(2))-propen-1-yl]chinolin und die Verbindung Nr. 15 oder Nr. 18 durch die Verbindung Nr. 3 in gleicher Menge ersetzt wird und eine Polyesterfolie beschichtet wird.

12 Sekunden bildmäßige Belichtung entsprechend Beispiel 1 lassen die Farbe der Bildstellen von anfangs hellrot nach tiefviolett umschlagen.

Durch Überwischen mit dem Entwickler aus Beispiel 3 werden die Nichtbildstellen entfernt. Man erhält ein negatives Abbild der Vorlage.

Dieses Vorgehen eignet sich zur Herstellung von Farbfolien.

**Beispiel 9**

Eine Platte aus mechanisch aufgerauhtem Aluminium wird mit einer Schicht der folgenden Zusammensetzung, aufgeschleudert aus 10-%-iger Methylethylketon-Lösung, versehen:

48,3 Gt eines Epoxidharzes (aus Epichlorhydrin und Bisphenol A, Epoxyäquivalentgewicht 182 - 194),
48,3 Gt des Novolaks nach Beispiel 1,
2,9 Gt der Verbindung Nr. 4 und
0,5 Gt Kristallviolett.

45 Sekunden bildmäßige Belichtung entsprechend Beispiel 1 und 40 Sekunden Entwickeln mit dem Entwickler aus Beispiel 7 erzeugen ein negatives Abbild der Vorlage mit schleierfreien Nichtbildstellen.

Ersetzt man das Epoxidharz durch die gleiche Menge des angegebenen Novolaks, wird bei der Entwicklung zwar kurzzeitig ein negatives Bild sichtbar, die Entwicklerresistenz ist jedoch so gering, daß nach 30 Sekunden die gesamte Schicht vom Träger abgelöst ist.

**Beispiel 10**

Dicke Schichten eines positiv arbeitenden Fotoresists werden folgendermaßen hergestellt:
Eine Lösung aus

60 Gt Butanon,
30 Gt des Novolaks nach Beispiel 1,
8,58 Gt eines Polyacetals aus 2-Ethyl-hexanal und Pentan-1,5-diol,
0,12 Gt der Verbindung Nr. 7 und
1,3 Gt Polyvinylmethylether

trägt man mit Hilfe einer Zieh-Spiralrakel (wire bar Nr. 40) auf die gereinigte Kupferoberfläche des Verbundmaterials aus Beispiel 6 auf, läßt das Lösungsmittel durch zwölfstündige Lagerung bei Raumtemperatur weitgehend verdunsten und trocknet die Platte 15 Minuten unter Infrarotstrahlung bei 70°C nach.

Die 60 Sekunden unter einer Strichvorlage mit der Lichtquelle aus Beispiel 1 belichtete, 70 µm dicke Resistschicht läßt sich durch Ansprühen mit 0,8-%-iger wäßriger Natronlauge in 40 Sekunden entwickeln.

**Beispiel 11**

Eine Lösung aus

23,3 Gt des Novolaks nach Beispiel 1,
6,9 Gt des Bisorthoesters nach Beispiel 4 und
1,0 Gt der Verbindung Nr. 1 in
6,9 Gt Xylol,
6,9 Gt Butylacetat und
55,0 Gt 2-Ethoxy-ethylacetat

wird mit Hilfe einer Schleuder bei 4000 Umdrehungen je Minute auf eine Siliciumscheibe von 7,6 cm Durchmesser, die mit einer 1,0 µm dicken Oxidschicht versehen ist, aufgebracht. Nach 30 Minuten Trocknen bei 90°C im Umlufttrockenschrank beträgt die Resistschichtdicke 1,15 µm. Die beschichtete Siliciumscheibe wird

durch eine Maske in einem Kontaktbelichtungsgerät, bestückt mit einer Quecksilberhochdrucklampe, belichtet. 5,6 $mJ/cm^2$ genügen, um die belichteten Stellen der Resistschicht in 90 Sekunden mit dem Entwickler des Beispiels 1 herauslösen zu können.

## Patentansprüche

1. Lichtempfindliche Verbindungen der allgemeinen Formel I

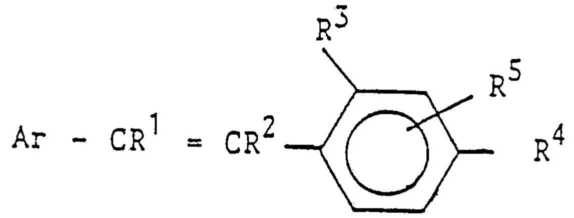

(I)

worin
$R^1$ und $R^2$ Wasserstoffatome oder Methylgruppen bedeuten,
$R^3$ und $R^4$ voneinander verschieden sind und jeweils ein Wasserstoffatom oder einen 4,6-Bis-trichlormethyl-s-triazin-2-ylrest bedeuten,
$R^5$ ein Wasserstoff- oder Halogenatom, einen Alkyl-, oder Alkoxyrest und
Ar Phenyl bedeutet, das 1- bis 3-fach durch Halogenatome, Alkyl-, Halogenalkyl-, Aralkyl-, Aryloxyalkyl-, Cycloalkyl-, Alkenyl-, Aryl- oder Aryloxyreste substituiert sein kann, wobei in den Alkylresten einzelne Methylengruppen durch O- oder S-Atome ersetzt, jeweils zwei dieser Reste unter Ausbildung eines 5- oder 6-gliedrigen Rings verknüpft sein können und die Substituenten insgesamt nicht mehr als 12 Kohlenstoffatome aufweisen, oder aber ein Naphthyl-, Acenaphthyl-, Di- oder Tetrahydronaphthyl-, Indanyl-, Anthryl-, Phenanthryl- Fluorenyl- oder Tetrahydrophenanthrylrest ist, der durch Halogenatome, Alkylreste mit 1 bis 3, Alkoxyreste mit 1 bis 4 oder Alkoxyalkylreste mit 3 bis 6 Kohlenstoffatomen substituiert sein kann.

2. Verbindungen nach Anspruch 1, dadurch gekennzeichnet, daß $R^1$, $R^2$, $R^3$ und $R^5$ Wasserstoffatome sind.

3. Lichtempfindliches Gemisch, enthaltend ein Bis-trichlormethyl-s-triazin (a) und eine Verbindung (b), die mit dem Lichtreaktionsprodukt des Triazins (a) unter Ausbildung eines Produktes zu reagieren vermag, das eine von (b) unterschiedliche Lichtabsorption oder Löslichkeit in einem Entwickler aufweist, dadurch gekennzeichnet, daß das Triazin (a) eine Verbindung der Formel I gemäß Anspruch 1 ist.

4. Lichtempfindliches Gemisch nach Anspruch 3, dadurch gekennzeichnet, daß die Verbindung (b) eine ethylenisch ungesättigte Verbindung ist, die eine durch freie Radikale ausgelöste Polymerisation einzugehen vermag.

5. Lichtempfindliches Gemisch nach Anspruch 3, dadurch gekennzeichnet, daß die Verbindung (b) mindestens eine durch Säure spaltbare C-O-C-Bindung enthält.

6. Lichtempfindliches Gemisch nach Anspruch 3, dadurch gekennzeichnet, daß die Verbindung (b) durch Säure zur kationischen Polymerisation angeregt zu werden vermag.

7. Lichtempfindliches Gemisch nach Anspruch 3, dadurch gekennzeichnet, daß die Verbindung (b) durch Säure vernetzbar ist.

8. Lichtempfindliches Gemisch nach Anspruch 3, dadurch gekennzeichnet, daß die Verbindung (b) durch Einwirkung von Säure ihren Farbton ändert.

9. Lichtempfindliches Gemisch nach Anspruch 3, dadurch gekennzeichnet, daß es die Verbindung der Formel I in einer Menge von 0,05 bis 10 Gew.-%, bezogen auf seine nichtflüchtigen Bestandteile, enthält.

10. Lichtempfindliches Gemisch nach Anspruch 3, dadurch gekennzeichnet, daß es zusätzlich ein wasserunlösliches polymeres Bindemittel enthält.

11. Lichtempfindliches Gemisch nach Anspruch 10, dadurch gekennzeichnet, daß das Bindemittel in wäßrig-alkalischen Lösungen löslich ist.

12. Verfahren zur Herstellung einer Verbindung der Formel I gemäß Anspruch 1, dadurch gekennzeichnet, daß man 1 mol einer Verbindung der Formel III

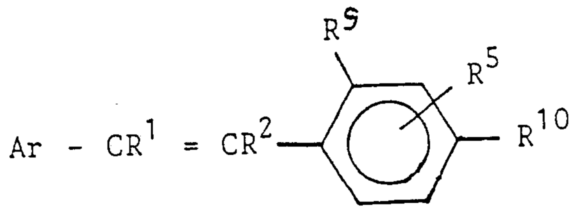

(III)

worin
$R^9$ und $R^{10}$ voneinander verschieden sind und jeweils ein Wasserstoffatom oder einen CN-Rest bedeuten und Ar, $R^1$, $R^2$ und $R^5$ die im Anspruch 1 angegebene Bedeutung haben,
und 2 - 8 mol Trichloracetonitril in Gegenwart von Halogenwasserstoff und Friedel-Crafts-Katalysatoren cotrimerisiert.

**Claims**

1. Light-sensitive compounds of the general formula I:

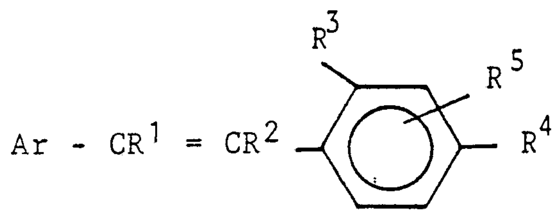

(I)

in which
$R^1$ and $R^2$ denote hydrogen atoms or alkyl groups,
$R^3$ and $R^4$ are different from one another and each denote a hydrogen atom or a 4,6-bis-trichloromethyl-s-triazin-2-yl group,
$R^5$ denotes a hydrogen or halogen atom or an alkyl or alkoxy group, and
Ar is a phenyl group which may carry 1 to 3 substituents selected from the group including halogen atoms, alkyl, halogenoalkyl, aralkyl, aryl-oxyalkyl, cycloalkyl, alkenyl, aryl or aryloxy groups, whereby in the alkyl groups individual methylene groups can be replaced by oxygen or sulphur atoms and two of these groups can each be linked to form a 5- or 6-member ring, and the maximum total number of carbon atoms contained in the substituents is 12; or is a naphthyl, acenaphthyl, di- or tetrahydronaphthyl, indanyl, anthryl, phenanthryl, fluorenyl or tetrahydrophenanthryl group which can be substituted by halogen atoms, by alkyl groups having 1 to 3 carbon atoms, by alkoxy groups having 1 to 4 carbon atoms or by alkoxy-alkyl groups having 3 to 6 carbon atoms.

2. Compounds as claimed in Claim 1, wherein $R^1$, $R^2$, $R^3$, and $R^5$ are hydrogen atoms.

3. Light-sensitive mixture containing a bis-trichloromethyl-s-triazine (a) and a compound (b) which is capable of reacting with the photoreaction product of the triazine (a) in such a way that a product is formed, the light absorption or solubility in developer of which is different from that of (b), characterized in that the triazine (a) is a compound of the general formula 1 as claimed in Claim 1.

4. Light-sensitive mixture as claimed in Claim 3, wherein the compound (b) is an ethylenically unsaturated compound which is capable of undergoing a polymerization reaction initiated by free radicals.

5. Light-sensitive mixture as claimed in Claim 3, wherein the compound (b) contains at least one acid-cleavable C-O-C-bond.

6. Light-sensitive mixture as claimed in Claim 3, wherein the compound (b) can be induced by an acid to undergo a cationic polymerization.

7. Light-sensitive mixture as claimed in Claim 3, wherein the compound (b) can be cross-linked by an acid.

8. Light-sensitive mixture as claimed in Claim 3, wherein the color of the compound (b) changes by the action of an acid.

9. Light-sensitive mixture as claimed in Claim 3, wherein the compound of the general formula 1 is contained in an amount of between 0.05 and 10 % by weight, relative to its non-volatile constituents.

10. Light-sensitive mixture as claimed in Claim 3, wherein additionally a water-insoluble polymeric binder is contained.

11. Light-sensitive mixture as claimed in Claim 10, wherein the binder is soluble in aqueous-alkaline solutions.

12. Process for the production of a compound of the general formula I as claimed in Claim 1, wherein 1 mole of a compound of the general formula III:

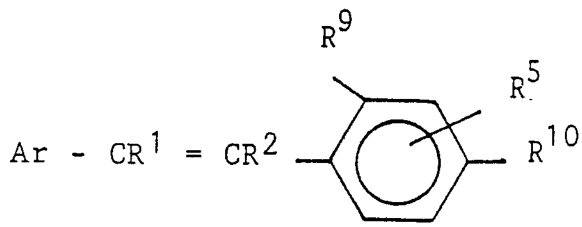

(III)

in which

$R^9$ and $R^{10}$ are different from one another and each denote a hydrogen atom or a CN-group, and

Ar, $R^1$, $R^2$, and $R^5$ have the same meaning as in Claim 1, and 2 to 8 moles of trichloroacetonitrile are cotrimerized in the presence of hydrogen halide and Friedel-Crafts catalysts.

## Revendications

1. Composés photosensibles de formule générale I

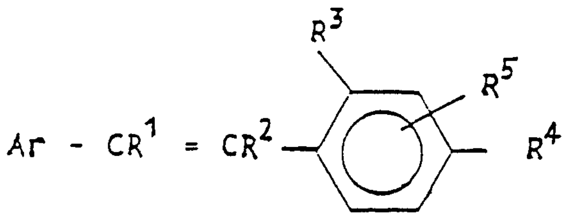

(I)

dans laquelle

$R^1$ et $R^2$ représentent des atomes d'hydrogène ou des groupes méthyle;

$R^3$ et $R^4$ sont différents l'un de l'autre et représentent chacun un atome d'hydrogène ou un radical 4,6-bis-trichlorométhyl-s-triazine-2-yle;

$R^5$ représente un atome d'hydrogène ou d'halogène ou un radical alkyle ou alcoxy; et

Ar représente un radical phényle qui peut être 1 à 3 fois substitué par des atomes d'halogène ou par des groupes alkyle, halogénoalkyle, aralkyle, aryloxyalkyle, cycloalkyle, alcényle, aryle ou aryloxy, des groupes méthylène individuels dans les radicaux alkyle pouvant être remplacés par des atomes d'O ou de S, deux de ces radicaux pouvant être liés avec formation d'un cycle à 5 ou 6 chaînons, et les substituants ne comportant au total pas plus de 12 atomes de carbone, ou bien est un radical naphtyle, acénaphtyle, di- ou tétrahydronaphtyle, indanyle, anthryle, phénanthryle, fluorényle ou tétrahydrophénanthryle, qui peut être substitué par des atomes d'halogène ou par des groupes alkyle ayant de 1 à 3 atomes de carbone, des groupes alcoxy ayant de 1 à 4 atomes de carbone ou des groupes alcoxyalkyle ayant de 3 à 6 atomes de carbone.

2. Composés selon la revendication 1, caractérisés en ce que $R^1$, $R^2$, $R^3$ et $R^5$ sont des atomes d'hydrogène.

3. Mélange photosensible contenant une bis-trichlorométhyl-s-triazine (a) et un composé (b) qui est capable de réagir avec le produit de photoréaction de la triazine (a) pour donner un produit dont l'absorption de la lumière ou la solubilité dans un révélateur est différente de celle de (b), caractérisé en ce que la triazine (a) est un composé de formule I selon la revendication 1;

4. Mélange photosensible selon la revendication 3, caractérisé en ce que le composé (b) est un composé à insaturation éthylénique, qui est capable de subir une polymérisation déclenchée par des radicaux libres.

5. Mélange photosensible selon la revendication 3, caractérisé en ce que le composé (b) contient au moins une liaison C-O-C pouvant être rompue par un acide.

6. Mélange photosensible selon la revendication 3, caractérisé en ce que le composé (b) est capable d'être induit par un acide à la polymérisation cationique.

7. Mélange photosensible selon la revendication 3, caractérisé en ce que le composé (b) est réticulable par un acide.

8. Mélange photosensible selon la revendication 3, caractérisé en ce que la couleur du composé (b) est modifiée sous l'effet d'un acide.

9. Mélange photosensible selon la revendication 3, caractérisé en ce qu'il contient le composé de formule I en une quantité de 0,05 à 10 % en poids, par rapport à ses composants non volatils.

10. Mélange photosensible selon la revendication 3, caractérisé en ce qu'il contient en outre un liant polymère insoluble dans l'eau.

11. Mélange photosensible selon la revendication 10, caractérisé en ce que le liant est soluble dans des solutions aqueuses alcalines.

12. Procédé pour la préparation d'un composé de formule I selon la revendication 1, caractérisé en ce que l'on co-trimérise 1 mole d'un composé dé formule III:

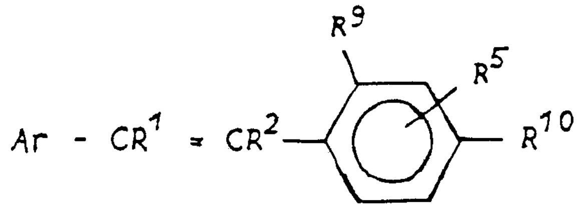

(III)

dans laquelle

$R^9$ et $R^{10}$ sont différents l'un de l'autre et représentent chacun un atome d'hydrogène ou un radical CN, et

Ar, $R^1$, $R^2$ et $R^5$ ont les significations données dans la revendication 1,

et 2 à 8 moles de trichlororacétonitrile, en présence d'un hydracide halogéné et de catalyseurs de Friedel et Crafts.